(19) Europäisches Patentamt
European Patent Office
Office européen des brevets

(11) Veröffentlichungsnummer : **0 007 024**
**B1**

(12) # EUROPÄISCHE PATENTSCHRIFT

(45) Veröffentlichungstag der Patentschrift : **17.03.82**

(51) Int. Cl.³ : **C 07 D239/14**, C 07 D405/12, C 07 D409/12, A 61 K 31/505

(21) Anmeldenummer : **79102051.4**

(22) Anmeldetag : **21.06.79**

(54) **Neue substituierte 2-Phenylamino-1,3-tetrahydropyrimidine-(2), deren Säureadditionssalze, diese enthaltende Arzneimittel und Verfahren zur Herstellung derselben.**

(30) Priorität : **19.07.78 DE 2831600**

(43) Veröffentlichungstag der Anmeldung : **23.01.80 (Patentblatt 80/02)**

(45) Bekanntmachung des Hinweises auf die Patenterteilung : **17.03.82 Patentblatt 82/11**

(84) Benannte Vertragsstaaten : **AT BE CH DE FR GB IT LU NL SE**

(56) Entgegenhaltungen : **FR - A - 2 212 143**

(73) Patentinhaber : **C.H. BOEHRINGER SOHN**
**Postfach 200**
**D-6507 Ingelheim am Rhein (DE)**

(72) Erfinder : **Stähle, Helmut, Dr.**
**Rotweinstrasse 23**
**D-6507 Ingelheim (DE)**
Erfinder : **Köppe, Herbert, Dr.**
**Neuweg 72**
**D-6507 Ingelheim (DE)**
Erfinder : **Kummer, Werner, Dr.**
**Georg-Scheuing-Strasse 15**
**D-6507 Ingelheim (DE)**
Erfinder : **Kobinger, Walter, Prof. Dr.**
**Beighofergasse 27**
**A-1120 Wien (AT)**
Erfinder : **Lillie, Christian, Dr. Mag.**
**Hansi-Niese-Weg 12**
**A-1130 Wien (AT)**
Erfinder : **Pichler, Ludwig, Dr.**
**Gusshausstrasse 24**
**A-1040 Wien (AT)**

**EP 0 007 024 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Neue substituierte 2-Phenylamino-1,3-tetrahydropyrimidine-(2) deren Säureadditionssalze, diese enthaltende Arzneimittel und Verfahren zur Herstellung derselben

In FR-A 2 212 143 werden Cyclopropylmethyl-aminoheterocyclen beschrieben, die blutdrucksenkende und ZNS-dämpfende Wirkungen besitzen. Darüber hinaus wird auch über eine bradycarde Wirkung berichtet. Die Bradycardie dieser bekannten Verbindungen wird reflektorisch ausgelöst. Es wurde gefunden, daß neue, am Brückenstickstoff-atom substituierte 2-Phenylamino-1,3-tetrahydropyrimidine-(2) im Gegensatz zu den Derivaten der FR-A 2 212 143 eine bradycarde Wirkung durch direkten Angriff auf den Sinusknoten des Herzens hervorrufen.

Die Erfindung betrifft neue substituierte 2-Phenylamino-1,3-tetrahydropyrimidine der allgemeinen Formel

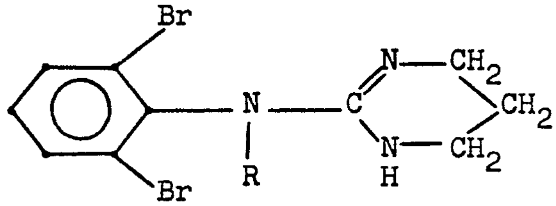

(I)

sowie deren physiologisch verträgliche Säureadditionssalze mit wertvollen therapeutischen Eigenschaften.

In der Formel I bedeutet

R den Rest —$CH_2$—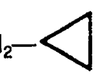, —$CH_2$—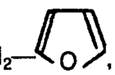, —$CH_2$—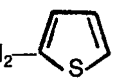, $CH_2$—CH=$CH_2$, —$CH_2$—CH=CH—$CH_3$, —$CH_2$—C($CH_3$)=$CH_2$

Die Herstellung der Verbindungen der Formel I erfolgt durch :

a) Umsetzung von 2-(2,6-Dibromphenylimino)-1,3-diazacyclohexan der Formel

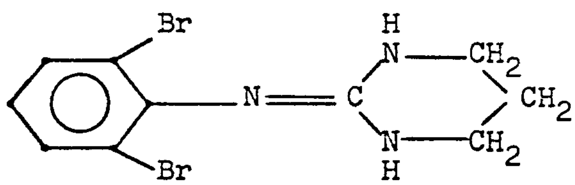

(II)

mit einem Halogenid der Formel

Hal———R (III)

worin Hal ein Chlor-, Brom- oder Jodatom bedeutet und R die obige Bedeutung hat ; oder

b) Umsetzung einer Verbindung der allgemeinen Formel

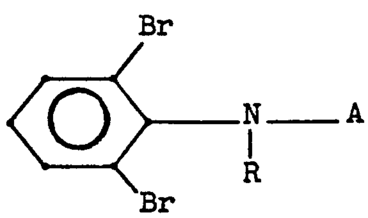

(IV)

in der R wie oben angegeben definiert ist und A eine Cyanogruppe oder den Rest

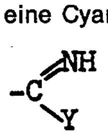

bedeutet, wobei Y eine Alkoxy- oder Alkylthiogruppe mit bis zu 4 C-Atomen oder eine Sulfhydril- oder Aminogruppe darstellt, mit 1,3-Diaminopropan oder dessen Säureadditionssalzen.

Bei der Alkylierung des 2-Arylimino-tetrahydropyrimidine der Formel II nach Verfahren a) erfolgt die Substitution ausschließlich am Brückenstickstoffatom. Bei der Umsetzung nach den Verfahren b) ist die

Konstitution der Endverbindungen durch die Synthese festgelegt. Die Position der Substituenten läßt sich außer durch die Synthese auch durch die NMR-Spektroskopie festlegen. (Vgl. H. Stähle und K. -H. Pook, Liebigs Ann. Chem. 751, 159 ff (1971)).

Die Umsetzung nach Verfahren a) erfolgt zweckmäßigerweise durch Erhitzen der Reaktionspartner — vorzugsweise in Gegenwart eines polaren oder unpolaren organischen Lösungsmittels — auf Temperaturen von etwa 50 bis 150 °C. Die speziellen Reaktionsbedingungen hängen in starkem Maße von der Reaktivität der Umsetzungsteilnehmer ab. Es empfiehlt sich, bei der Alkylierung das Halogenid im Überschuß anzuwenden und die Umsetzung in Gegenwart eines säurebindenden Mittels durchzuführen.

Bei dem Verfahren nach b) ist es erforderlich, bei erhöhter Temperatur, zwischen 60 °C und 180 °C zu arbeiten. Lösungsmittel sind nicht erforderlich. Es ist zweckmäßig, das als Reaktionspartner verwendete 1,3-Diaminopropan bzw. dessen Säureadditionssalz im Überschuß anzuwenden.

Ausgangsverbindungen der Formel II sind z.B. in den belgischen Patenten Nr. 623 305, 687 657 und 705 944 beschrieben.

Ausgangsverbindungen der Formel III lassen sich durch Halogenierung der zugrundeliegenden primären Alkohole darstellen.

Verbindungen der Formel IV erhält man ausgehend von Anilinen, durch Umsetzung mit Verbindungen der Formel III und nachfolgende Reaktion der dabei entstehenden sekundären Amine, mit Cyanaten oder Thiocyanaten wobei Harnstoffe, bzw. Thioharnstoffe gebildet werden. Harnstoffe und Thioharnstoffe können dann weiter mit Alkylierungsmitteln in entsprechende Isouroniumsalze bzw. Isothiouroniumsalze überführt werden. Aus diesen Säureadditionsverbindungen lassen sich mit Basen die entsprechenden Isoharnstoffe bzw. Isothioharnstoffe gewinnen. Durch Wasserabspaltung aus Harnstoffen bzw. $H_2S$-Abspaltung aus Thioharnstoffen mittels Blei- oder Quecksilbersalzen gelangt man zu Cyanamiden, an die Ammoniak unter Bildung von Guanidinen angelagert werden kann.

Die erfindungsgemäßen 2-Phenylamino-tetrahydropyrimidine-(2) der allgemeinen Formel I können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neuen Verbindungen der allgemeinen Formel I und deren Säureadditionssalze wirken sehr stark bradycard und sind daher zur Therapie von Coronarerkrankungen geeignet. Die Beeinflussung der Herzfrequenz wurde an Kaninchen und an Spinalratten sowie an intakten narkotisierten Ratten untersucht.

Die Dosierung liegt bei 0,1 bis 80 mg, vorzugsweise 1 bis 30 mg.

Die Verbindungen der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung, ohne sie zu beschränken.

## Beispiel 1

2-[N-(Cyclopropylmethyl)-N-(2,6-dibromphenyl)-amino]-1,3-tetrahydropyrimidin-(2)

7,7 g (0,02 Mol) 2-(2,6-Dibromphenylimino)-1,3-diaza-cyclohexan werden zusammen mit 2,26 g (125 %) Chlormethylcyclopropan und 5 ml Triäthylamin in 25 ml getrocknetem Toluol 18 Stunden lang im Rohr auf 125 °C erhitzt. Nach Abziehen des Lösungsmittels im Vakuum wird in verdünnter Salzsäure gelöst und die saure Lösung mehrmals mit Äther extrahiert (Ätherextrakte werden verworfen). Nach Behandeln mit Aktivkohle wird die salzsaure Lösung bei aufsteigenden pH-Werten (Alkalisieren mit verdünnter Natronlauge) fraktioniert mit Äther extrahiert. Die dünnschicht-chromatografisch einheitlichen Ätherfraktionen werden vereinigt, über $CaSO_4$ getrocknet und der Äther im Vakuum abgezogen. Es verbleiben 0,35 g des Tetrahydropyrimidinderivats, welches nach Anrühren mit Petroläther durch-kristallisiert. Ausbeute : 4,5 % der Theorie, Schmelzpunkt : 128-130 °C.

Analog dem vorstehenden Beispiel wurden die folgenden Verbindungen hergestellt. Die Schmelzpunkte beziehen sich auf die freien Basen. Anderenfalls ist die Salzform angegeben.

| Beispiel Nr. | R. | Fp °C | Ausbeute % der Theorie |
|---|---|---|---|
| 2 | $—CH_2—$ 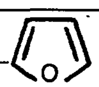 | 245-246 (Hydrobromid) | 20,2 |

(Fortsetzung)

| Beispiel Nr. | R. | Fp °C | Ausbeute % der Theorie |
|---|---|---|---|
| 3 | $-CH_2-$ 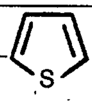 | 243-244 (Hydrobromid) | 12,4 |
| 4 | $-CH_2-CH=CH_2$ | 118-120 | 65,5 |
| 5 | $-CH_2-CH=CH-CH_3$ | 86-88 | 43,4 |
| 6 | $-CH_2-C(CH_3)=CH_2$ | 87-89 | 23,5 |

## Formulierungsbeispiele

Beispiel A : Tabletten

| | |
|---|---|
| Wirkstoff gemäß Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

Herstellung :

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpreßt, die dann in üblicher Weise mit Hilfe von Zucker, Talkum und Gummi arabicum dragiert werden.

Beispiel B : Ampullen

| | | |
|---|---|---|
| Wirkstoff gemäß Erfindung | | 1,0 mg |
| Natriumchlorid | | 18,0 mg |
| dest. Wasser | ad | 2,0 ml |

Herstellung :

Wirkstoff und Natriumchlorid werden in Wasser gelöst und unter Stickstoff in Glasampullen abgefüllt.

Beispiel C : Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

**Ansprüche** (für die Vertragsstaaten : BE, CH, DE, FR, GB, IT, LU, NL, SE)

1. Substituierte 2-Phenylamino-1,3-tetrahydropyrimidine-(2) der allgemeinen Formel

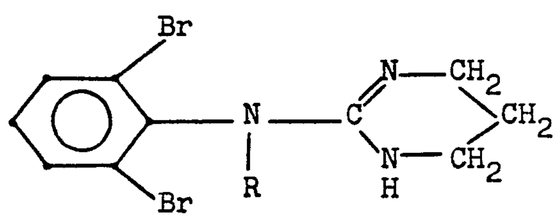

(I)

worin

R den Rest —$CH_2$—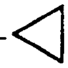, —$CH_2$—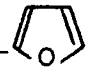, —$CH_2$—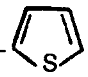, —$CH_2$—CH = $CH_2$, —$CH_2$—CH = CH—$CH_3$, —$CH_2$—C($CH_3$) = $CH_2$

bedeutet sowie deren Säureadditionssalze.

2. Verfahren zur Herstellung von substituierten 2-Phenylamino-1,3-tetrahydropyrimidinen-(2) der allgemeinen Formel I nach Anspruch 1 und von deren Säureadditionssalzen, dadurch gekennzeichnet, daß man

a) 2-(2,6-Dibromphenylimino)-1,3-diazacyclohexan der Formel

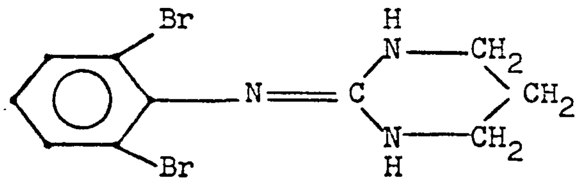

(II)

mit einem Halogenid der allgemeinen Formel

$$Hal—R \quad (III)$$

worin Hal ein Chlor-, Brom- oder Jodatom bedeutet und R die genannte Bedeutung hat, umsetzt oder

b) eine Verbindung der allgemeinen Formel

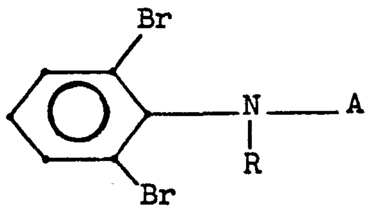

(IV)

in der R wie oben angegeben definiert ist und A eine Cyanogruppe oder den Rest

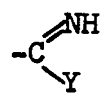

bedeutet, wobei Y eine Alkoxy- oder Alkylthiogruppe mit bis zu 4 Kohlenstoffatomen oder eine Sulfhydryl- oder Aminogruppe darstellt, mit 1,3-Diaminopropan oder dessen Säureadditionssalzen umsetzt, und daß man gegebenenfalls das nach einem der Verfahren erhaltene Endprodukt in ein Säureadditionssalz überführt.

3. Verfahren nach Anspruch 2 a) dadurch gekennzeichnet, daß man die Umsetzung bei Temperaturen von etwa 50 bis 150 °C durchführt.

4. Verfahren nach Anspruch 2 b) dadurch gekennzeichnet, daß man die Umsetzung bei Temperaturen von 60 bis 180 °C durchführt.

5. Verfahren nach Anspruch 2 a) und/oder 3, dadurch gekennzeichnet, daß man die Umsetzung in Gegenwart eines polaren oder unpolaren organischen Lösungsmittels durchführt.

6. Verfahren nach Anspruch 2 a) 3 und/oder 5, dadurch gekennzeichnet, daß man die Umsetzung in Gegenwart eines säurebindenden Mittels durchführt.

7. Pharmazeutische Zubereitungen, dadurch gekennzeichnet, daß sie als Wirkstoff eine oder mehrere Verbindungen der allgemeinen Formel I nach Anspruch 1 oder deren Säureadditionssalze enthalten.

8. Verfahren zur Herstellung von Arzneimitteln nach Anspruch 6, dadurch gekennzeichnet, daß man eine oder mehrere Verbindungen der allgemeinen Formel I nach Anspruch 1 oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

9. Verbindungen der allgemeinen Formel I nach Anspruch 1 oder deren physiologisch verträgliche Säureadditionssalze zur Verwendung bei der Behandlung von Tachycardien.

**Ansprüche** (für den Vertragsstaat AT)

1. Verfahren zur Herstellung von substituierten 2-Phenylamino-1,3-tetrahydropyrimidinen-(2) der allgemeinen Formel

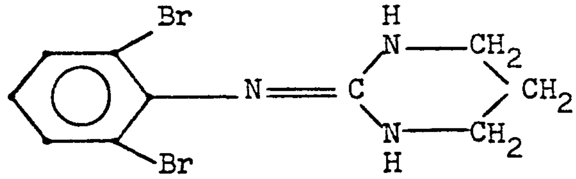

(I)

worin

R den Rest $—CH_2—$ 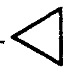 , $—CH_2—$ 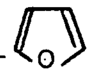 , $—CH_2—$ 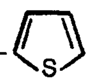 , $—CH_2—CH = CH_2$, $—CH_2—CH=CH—CH_3$, $—CH_2—C(CH_3)=CH_2$

bedeutet sowie von deren Säureadditionssalzen, dadurch gekennzeichnet, daß man

a) 2-(2,6-Dibromphenylimino)-1,3-diazacyclohexan der Formel

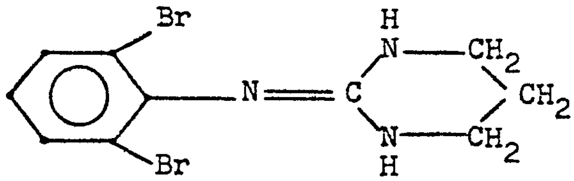

(II)

mit einem Halogenid der allgemeinen Formel

Hal——R (III)

worin Hal ein Chlor-, Brom- oder Jodatom bedeutet und R die genannte Bedeutung hat, umsetzt oder

b) eine Verbindung der allgemeinen Formel

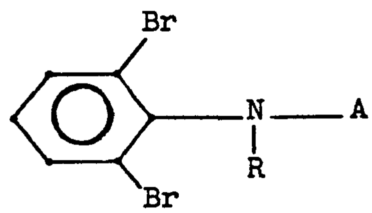

(IV)

in der R wie oben angegeben definiert ist und A eine Cyanogruppe oder den Rest

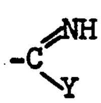

bedeutet, wobei Y eine Alkoxy- oder Alkylthiogruppe mit bis zu 4 Kohlenstoffatomen oder eine Sulfhydryl- oder Aminogruppe darstellt, mit 1,3 Diaminopropan oder dessen Säureadditionssalzen umsetzt, und daß man gegebenenfalls das nach einem der Verfahren erhaltene Endprodukt in ein Säureadditionssalz überführt.

2. Verfahren nach Anspruch 1 a) dadurch gekennzeichnet, daß man die Umsetzung bei Temperaturen von etwa 50 bis 150 °C durchführt.

3. Verfahren nach Anspruch 1 b) dadurch gekennzeichnet, daß man die Umsetzung bei Temperaturen von 60 bis 180 °C durchführt.

4. Verfahren nach Anspruch 1 a) und/oder 2, dadurch gekennzeichnet, daß man die Umsetzung in Gegenwart eines polaren oder unpolaren organischen Lösungsmittels durchführt.

5. Verfahren nach Anspruch 1 a), 2 und/oder 4, dadurch gekennzeichnet, daß man die Umsetzung in Gegenwart eines säurebindenden Mittels durchführt.

6. Verfahren zur Herstellung von Arzneimitteln, dadurch gekennzeichnet, daß man eine oder mehrere Verbindungen der allgemeinen Formel I nach Anspruch 1 oder deren Säureadditionssalze mit

üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

**Claims** (for the contracting states BE, CH, DE, FR, GB, IT, LU, NL, SE)

1. Substituted 2-phenylamino-1,3-tetrahydropyrimidines-(2) of general formula

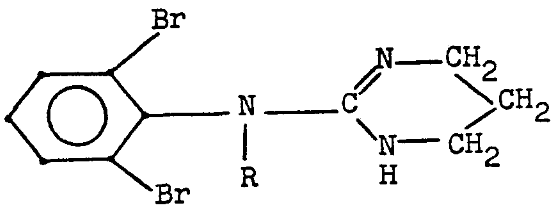

(I)

wherein

R represents the group $—CH_2—$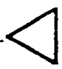, $—CH_2—$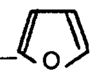, $—CH_2—$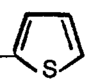, $—CH_2—CH=CH_2$, $—CH_2—CH = CH—CH_3$ or $—CH_2—C(CH_3) = CH_2$

and the acid addition salts thereof.

2. Process for preparing substituted 2-phenylamino-1,3-tetrahydropyrimidines-(2) of general formula I as claimed in claim 1 and the acid addition salts thereof, characterised in that

a) 2-(2,6-dibromophenylimino)-1,3-diazacyclohexane of formula

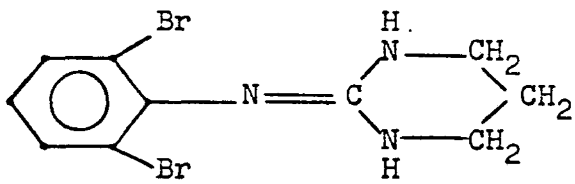

(II)

is reacted with a halide of general formula

Hal———R (III)

wherein Hal represents a chlorine, bromine or iodine atom and R is a hereinbefore defined, or

b) a compound of general formula

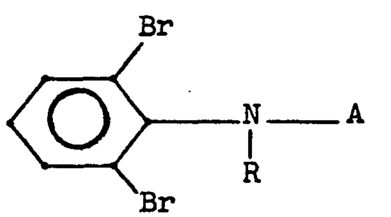

(IV)

wherein R is as hereinbefore defined and A represents a a cyano group or the group

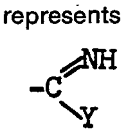

wherein Y represents an alkoxy or alkylthio group with up to 4 carbon atoms or a mercapto or amino group, is reacted with 1,3-diamino-propane or the acid addition salts thereof, and, if desired, the end product obtained according to one of the processes is converted into an acid addition salt.

3. Process as claimed in claim 2 (a), characterised in that the reaction is effected at temperatures of about 50 to 150 °C.

4. Process as claimed in claim 2 (b), characterised in that the reaction is effected at temperatures of from 60 to 180 °C.

5. Process as claimed in claim 2 (a) and/or 3, characterised in that the reaction is effected in the presence of a polar or non-polar organic solvent.

6. Process as claimed in claim 2 (a), 3 and/or 5, characterised in that the reaction is effected in the presence of an acid-binding agent.

7. Pharmaceutical preparations, characterised in that they contain as active ingredient one or more compounds of general formula I as claimed in claim 1 or the acid addition salts thereof.

8. Process for preparing pharmaceutical compositions as claimed in claim 6, characterised in that one or more compounds of general formula I as claimed in claim 1 or the acid addition salts thereof are processed with conventional galenic excipients, carriers, disintegrants or lubricants or substances for obtaining delayed release, to form tablets, capsules, suppositories, solutions or powders.

9. Compounds of general formula I as claimed in claim 1 or the physiologically acceptable acid addition salts thereof for use in the treatment of tachycardia.

**Claims** (for the contracting state AT).

1. Process for preparing substituted 2-phenylamino-1, 3-tetrahydropyrimidines-(2) of general formula

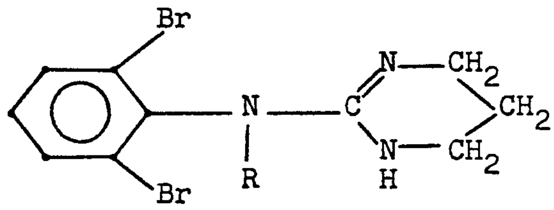

(I)

wherein

R represents the group $—CH_2—$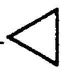, $—CH_2—$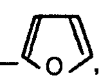, $—CH_2—$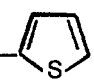, $—CH_2—CH=CH_2$, $—CH_2—CH=CH—CH_3$ or $—CH_2—C(CH_3)=CH_2$

and the acid addition salts thereof, characterised in that

a) 2-(2,6-dibromophenylimino)-1,3-diazacyclohexane of formula

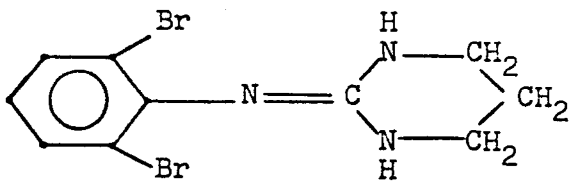

(II)

is reacted with a halide of general formula

Hal——R (III)

wherein Hal represents a chlorine, bromine or iodine atom and R is as hereinbefore defined, or

b) a compound of general formula

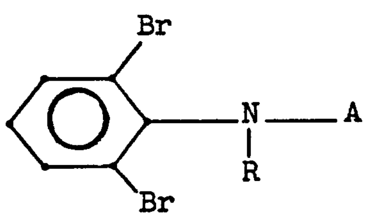

(IV)

wherein R is as hereinbefore defined and A represents a cyano group or the group

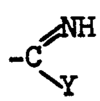

wherein Y represents an alkoxy or alkylthio group with up to 4 carbon atoms or a mercapto or amino group, is reacted with 1,3-diaminopropane or the acid addition salts thereof, and, if desired, the end product obtained according to one of these processes is converted into an acid addition salt.

2. Process as claimed in claim 1 (a), characterised in that the reaction is effected at temperatures of about 50 to 150 °C.

3. Process as claimed in claim 1 (b), characterised in that the reaction is effected at temperatures of from 60 to 180 °C.

4. Process as claimed in claim 1 (a), and/or 2, characterised in that the reaction is effected in the presence of a polar or non-polar organic solvent.

5. Process as claimed in claim 1 (a), 2 and/or 4, characterised in that the reaction is effected in the presence of an acid-binding agent.

6. Process for preparing pharmaceutical compositions, characterised in that one or more compounds of general formula I according to claim 1 or the acid addition salts thereof are processed with conventional galenic excipients, carriers, disintegrants or lubricants or substances for obtaining delayed release, to form tablets, capsules, suppositories, solutions or powders.

**Revendications** (pour les Etats contractants : BE, CH, DE, FR, GB, IT, LU, NL, SE)

1. 2-phénylamino-1,3-tétrahydropyrimidines-(2) substituées de formule générale

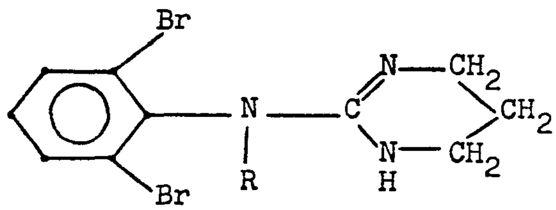

(I)

où

R signifie le radical —$CH_2$— 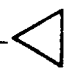 , —$CH_2$— 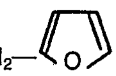 , —$CH_2$— 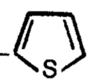 , —$CH_2$—$CH = CH_2$,

—$CH_2$—$CH$=$CH$—$CH_3$, —$CH_2$—$C(CH_3)$=$CH_2$

ainsi que leurs sels d'addition avec des acides.

2. Procédé pour la préparation de 2-phénylamino-1,3-tétrahydropyrimidines-(2) substituées de formule générale I selon la revendication 1 et de leurs sels d'addition avec des acides, caractérisé en ce que

a) on fait réagir le 2-(2,6-dibromophénylimino)-1,3-diazacyclohexane de formule

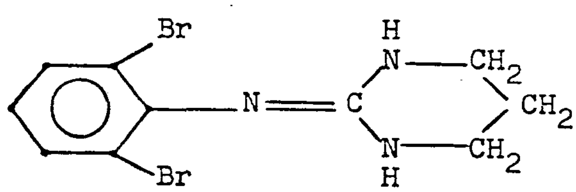

(II)

avec un halogénure de formule générale

Hal——— R (III)

où Hal signifie un atome de chlore, de brome ou d'iode et R a la signification indiquée, ou

b) on fait réagir un composé de formule générale

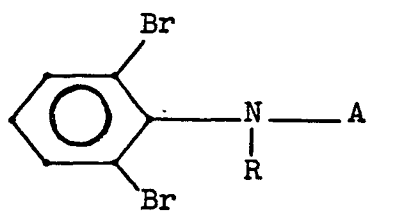

(IV)

dans laquelle R est défini comme indiqué plus haut et A signifie un groupe cyano ou le radical

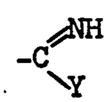

où Y représente un groupe alcoxy ou alcoylthio avec jusqu'à 4 atomes de carbone ou un groupe sulfhydryle ou amino, avec le 1,3-diaminopropane ou ses sels d'addition avec des acides, et en ce qu'on

transforme éventuellement le produit final obtenu selon l'un des procédés, en un sel d'addition avec un acide.

3. Procédé selon la revendication 2 a), caractérisé en ce qu'on effectue la réaction à des températures d'environ 50 à 150 °C.

4. Procédé selon la revendication 2 b), caractérisé en ce qu'on effectue la réaction à des températures de 60 à 180 °C.

5. Procédé selon la revendication 2 a) et/ou 3, caractérisé en ce qu'on effectue la réaction en présence d'un solvant organique polaire ou non polaire.

6. Procédé selon la revendication 2 a), 3 et/ou 5, caractérisé en ce qu'on effectue la réaction en présence d'un agent fixant les acides.

7. Préparations pharmaceutiques, caractérisées en ce qu'elles contiennent en tant que substance active un ou plusieurs composés de formule générale I selon la revendication 1 ou leurs sels d'addition avec des acides.

8. Procédé pour la préparation de médicaments selon la revendication 7, caractérisé en ce qu'on formule un ou plusieurs composés de formule générale I selon la revendication 1 ou leurs sels d'addition avec des acides avec des adjuvants, excipients, désagrégeants ou lubrifiants ou respectivement substances pour obtenir un effet retard galéniques habituels, en comprimés, capsules, suppositoires, solutions ou poudres.

9. Composés de formule générale I selon la revendication 1 ou leurs sels d'addition physiologiquement supportables avec des acides pour l'utilisation dans le traitement des tachycardies.

**Revendications** (pour l'Etat contractant AT)

1. Procédé pour la préparation de 2-phénylamino-1,3-tétrahydropyrimidines-(2) substituées de formule générale

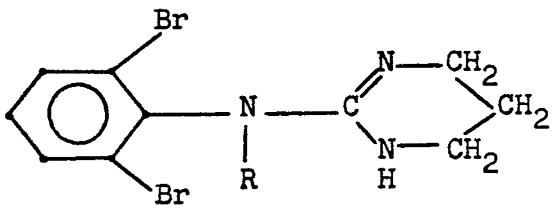

(I)

où

R signifie le radical $—CH_2—$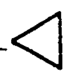, $—CH_2$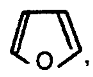, $—CH_2—$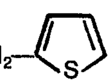, $—CH_2—CH=CH_2$, $—CH_2—CH=CH—CH_3$, $—CH_2—C(CH_3)=CH_2$

ainsi que de leurs sels d'addition avec des acides, caractérisé en ce que

a) on fait réagir du 2-(2,6-dibromophénylimino)-1,3-diazacyclohexane de formule

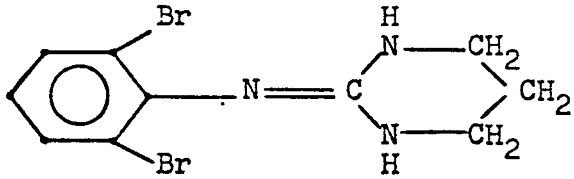

(II)

avec un halogénure de formule générale

$$Hal—R \qquad (III)$$

où Hal signifie un atome de chlore, de brome ou d'iode et R a la signification indiquée, ou

b) on fait réagir un composé de formule générale

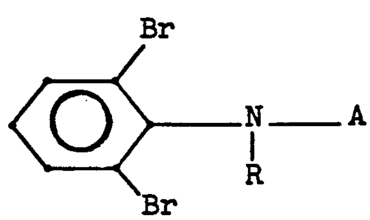

(IV)

dans laquelle R est défini comme indiqué plus haut et A signifie un groupe cyano ou le radical

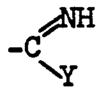

où Y représente un groupe alcoxy ou alcoylthio avec jusqu'à 4 atomes de carbone ou un groupe sulfhydryle ou amino, avec le 1,3-diaminopropane ou ses sels d'addition avec des acides, et en ce qu'on transforme éventuellement le produit final obtenu selon l'un des procédés en un sel d'addition avec un acide.

2. Procédé selon la revendication 1 a), caractérisé en ce qu'on effectue la réaction à des températures d'environ 50 à 150 °C.

3. Procédé selon la revendication 1 b), caractérisé en ce qu'on effectue la réaction à des températures de 60 à 180 °C.

4. Procédé selon la revendication 1 a), et/ou 2, caractérisé en ce qu'on effectue la réaction en présence d'un solvant organique polaire ou non polaire.

5. Procédé selon la revendication 1 a), 2 et/ou 4, caractérisé en ce qu'on effectue la réaction en présence d'un agent fixant les acides.

6. Procédé pour la préparation de médicaments, caractérisé en ce qu'on formule un ou plusieurs composés de formule générale I selon la revendication 1 ou ses sels d'addition avec des acides avec des adjuvants, excipients, désagrégeants ou lubrifiants ou respectivement substances pour obtenir un effet retard galéniques habituels, en comprimés, capsules, suppositoires, solutions ou poudres.